(19) Europäisches Patentamt
European Patent Office
Office européen des brevets

(11) Veröffentlichungsnummer: **0 104 423**
**B1**

(12) # EUROPÄISCHE PATENTSCHRIFT

(45) Veröffentlichungstag der Patentschrift: **21.05.86**

(21) Anmeldenummer: **83108250.8**

(22) Anmeldetag: **22.08.83**

(51) Int. Cl.[4]: **C 07 C 87/50,** C 07 D 213/36, C 07 D 213/74, C 07 C 93/14, C 07 C 93/04, C 07 C 149/42, C 07 C 91/44, C 07 C 85/02, C 07 C 89/00, A 61 K 31/04, A 61 K 31/44

(54) **2-Nitro-1,1-ethendiamine, Verfahren zu ihrer Herstellung sowie ihre Verwendung als Arzneimittel.**

(30) Priorität: **01.09.82 DE 3232462**

(43) Veröffentlichungstag der Anmeldung: **04.04.84 Patentblatt 84/14**

(45) Bekanntmachung des Hinweises auf die Patenterteilung: **21.05.86 Patentblatt 86/21**

(84) Benannte Vertragsstaaten: **AT BE CH DE FR GB IT LI NL SE**

(56) Entgegenhaltungen:
**GB - A - 1 421 792**
**GB - A - 1 564 502**

(73) Patentinhaber: **BAYER AG, Konzernverwaltung RP Patentabteilung, D-5090 Leverkusen 1 Bayerwerk (DE)**

(72) Erfinder: **Niemers, Ekkehard, Dr., In den Birken 51a, D-5600 Wuppertal 1 (DE)**
Erfinder: **Knorr, Andreas, Dr., Pahlkestrasse 15, D-5600 Wuppertal 1 (DE)**
Erfinder: **Garthoff, Bernward, Dr., Händelstrasse 22, D-4010 Hilden (DE)**

**EP 0 104 423 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue 2-Nitro-1,1-ethendiamine, mehrere Verfahren zu ihrer Herstellung sowie diese Verbindungen zur Verwendung als kreislaufbeeinflussende Arzneimittel, insbesondere als blutdrucksenkende Mittel.

Es ist bereits bekannt geworden, daß man 2-Nitro-1,1-ethendiamine erhält, wenn man 1-Nitro-2,2-bismethylthioethen mit Aminen zur Reaktion bringt (R. Gompper, H. Schaefer, Chem. Ber. 100, 591-604 (1967)).

Weiterhin ist bekannt, daß bestimmte 2-Nitro-1,1-ethen-diamine interessante pharmakologische Eigenschaften aufweisen (GB 1 564 502).

Die Erfindung betrifft 2-Nitro-1,1-ethendiamine der allgemeinen Formel I,

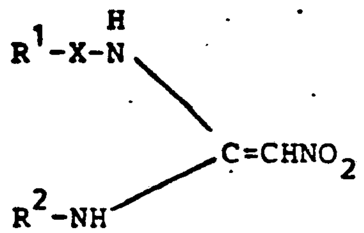

(I)

in welcher

$R^1$ für phenyl oder Naphthyl steht welche gegebenenfalls 1-bis 3-mal substituiert sind durch Halogen, Trifluormethyl, Nitro, Cyano, Hydroxy, Amino, Alkylamino, Dialkylamino, Alkyl, Alkoxy oder-Alkylmercapto mit jeweils 1 bis 4 C-Atomen in den Alkyl- und Alkoxy gruppen oder für Thienyl, Furyl, Pyridyl oder Pyrimidyl steht, welche gegebenenfalls ein- oder zweifach substituiert sind durch Hydroxy, Halogen, Alkyl oder Alkoxy mit jeweils 1 bis 4 C-Atomen,

$R^2$ für geradkettiges oder verzweigtes Alkyl mit bis zu 10 C-Atomen oder für cyclisches Alkyl mit bis zu 8 C-Atomen oder für geradkettiges, verzweigtes oder cyclisches Alkenyl mit 3 bis 8 C-Atomen steht, welche gegebenenfalls substituiert sind durch Alkoxy mit 1 bis 4 C-Atomen oder durch Cycloalkyl mit 3 bis 7 C-Atomen, oder für Phenyl oder Naphthyl steht, welches gegebenenfalls 1- bis 3-fach substituiert ist durch Halogen, Nitro, Cyano, Hydroxy, Trifluormethyl, Amino, Alkylamino, Dialkylamino, Alkyl, Alkoxy oder Alkylmercapto mit jeweils 1 bis 4 C-Atomen in den Alkoxy- und Alkylgruppen, und

X für eine einfache Bindung oder eine Methylengruppe steht, die gegebenenfalls 1- oder 2-fach durch Alkylreste mit 1 bis 4 Kohlenstoffatomen substituiert sein kann, wobei $R^2$ ungleich Phenyl oder substituiertes Phenyl ist, wenn X eine einfache Bindung bedeutet,

sowie ihre physiologisch verträglichen Säureadditionssalze und ihre isomeren Formen, insbesondere die cisund trans-Isomeren.

Es wurde gefunden, daß man die erfindungsgemäßen 2-Nitro-1,1-ethendiamine der allgemeinen Formel (I) erhält, wenn man

a) 2-Nitro-1-aminoethene der allgemeinen Formel (II)

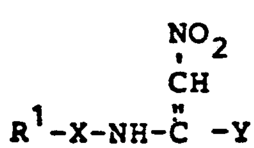

(II)

in welcher

Y für Alkylmercapto oder Alkylsulfinyl steht und

$R^1$ und X die oben angegebene Bedeutung haben,

mit Aminen der allgemeinen Formel (III)

$R^2$-$NH_2$ (III)

in welcher

$R^2$ die oben angegebene Bedeutung hat,

oder

b) 2-Nitro-1-aminoethene der allgemeinen Formel (IV)

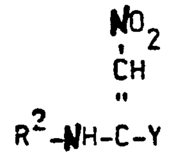

(IV)

in welcher
$R^2$ und Y die oben angegebene Bedeutung haben,
mit Aminen der allgemeinen Formel (V)
$R^1$-X-$NH_2$ (V)
in welcher
$R^1$ und X die oben angegebene Bedeutung haben,
gegebenenfalls in Gegenwart von inerten organischen Lösungsmitteln bei Temperaturen zwischen 0 und 180°C umsetzt.

Verwendet man 2-Nitro-1-anilino-1-methylthioethen und 1,2,2-Trimethylpropylamin als Ausgangsstoffe, so kann der Reaktionsablauf durch das folgende Formelschema wiedergegeben werden:

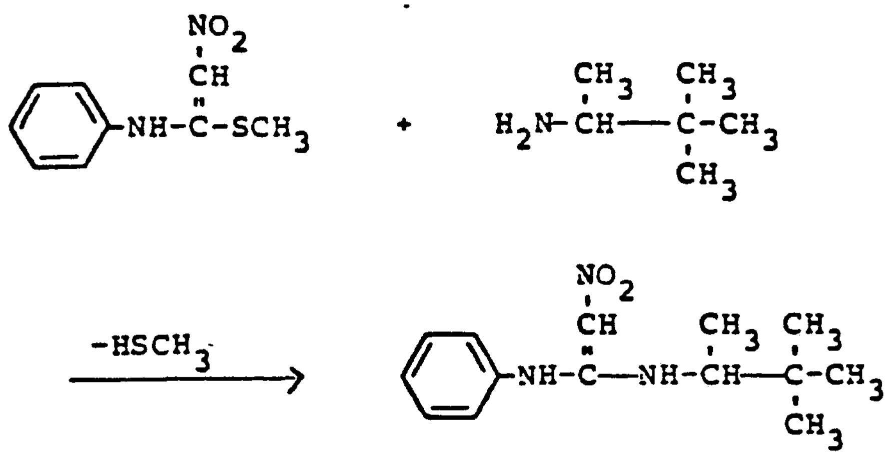


Die als Ausgangsmaterial verwendeten 2-Nitro-1-aminoethene der Formeln (II) und (IV) sind entweder literaturbekannt oder nach bekannten Verfahren herstellbar (vgl. Chem. Ber. 100, 591-604 (1967), Be 841 526).

Die Amine der allgemeinen Formeln (III) und (V) sind ebenfalls bekannt oder können nach bekannten Methoden hergestellt werden (vgl. Houben-Weyl X1/1 (1957)).

Bei dem Verfahren zur Herstellung der neuen 2-Nitro-1,1-ethendiamine kommen als Verdünnungsmittel alle inerten organischen Lösungsmittel in Frage. Hierzu gehören vorzugsweise Kohlenwasserstoffe, wie Hexan, Toluol, Xylol, Chlorkohlenwasserstoffe, wie Methylenchlorid, Chloroform, Chlorbenzol, Ether, wie Diethylether, Dioxan, Tetrahydrofuran, Alkohole, Pyridin, DMSO und DMF. Gelegentlich ist es jedoch von Vorteil kein Lösungsmittel zu verwenden, Die Reaktionstemperaturen können in einem größeren Bereich variiert werden. Im allgemeinen arbeitet man zwischen etwa 0°C und etwa 180°C vorzugsweise zwischen 20°C und 140°C. Die Umsetzung kann bei Normaldruck aber auch bei erhöhtem Druck durchgeführt werden.

Bei der Durchführung des erfindungsgemäßen Verfahrens setzt man vorzugsweise auf 1 Mol 2-Nitro-1-aminoethen 1 bis 5 Mol Amin.

Gelegentlich ist es von Vorteil, katalytische Mengen einer Säure, wie z.B. p-Toluolsulfonsäure, hinzuzufügen,

Falls nicht anders angegeben, bedeutet Alkyl in der vorliegenden Anmeldung geradkettiges oder verzweigtes Alkyl mit 1 bis 8 Kohlenstoffatomen insbesondere mit 1 bis 4 Kohlenstoffatomen. In der Definition des Substituenten $R^2$ in den allgemeinen Formeln (I), (III) und (IV) steht Alkyl vorzugsweise für geradkettiges oder verzweigtes Alkyl mit 1 bis 10, insbesondere mit 3 bis 8 Kohlenstoffatomen.

Beispielhaft seien genannt, n- und i-Propyl, n-, i- und t-Butyl, 1,1-Dimethylpropyl, 2,2-Dimethylpropyl, n-Pentyl, 1,2,2-Trimethylpropyl, 1,1-Dimethylbutyl, 1,1,2-Trimethyl-propyl, 1-Ethyl-1-methylpropyl, n-Hexyl, 1,1,3-Trimethylbutyl, 1,1-Diethylpropyl, n-Heptyl, n-Octyl.

In den allgemeinen Formeln I, II und V steht X unter anderem für eine Methylenbrücke, die ein- oder zweifach durch Alkylreste substituiert sein kann. Diese Alkylreste können geradkettig oder verzweigt sein und

vorzugsweise 1 bis 4 Kohlenstoffatome enthalten.

Wenn X eine einfache Bindung bedeutet, steht $R^2$ nicht für gegebenenfalls substituiertes Phenyl.

Von besonderem Interesse sind erfindungsgemäße Verbindungen der allgemeinen Formel (I), in welcher

$R^1$ für Phenyl steht, welches gegebenenfalls 1,2 oder 3 mal substituiert ist durch gleiche oder verschiedene Substituenten aus der Gruppe Halogen, Trifluormethyl, Alkyl, Alkoxy und Alkylmercapto mit jeweils 1 bis 4 Kohlenstoffatomen oder für Pyridyl oder Pyrimidyl steht,

$R^2$ für Phenyl steht, welches gegebenenfalls 1- oder 2-fach substituiert ist durch gleiche oder verschiedene Substituenten aus der Gruppe Halogen, Trifluormethyl, Alkyl oder Alkoxy mit jeweils 1 bis 4 Kohlenstoffatomen oder für geradkettiges, verzweigtes oder cyclisches Alkyl mit 1 bis 8 Kohlenstoffatomen oder für geradkettiges, verzweigtes oder cyclisches $C_3$-$C_8$-Alkenyl steht, welches gegebenenfalls substituiert ist durch Alkoxy mit 1 bis 4 Kohlenstoffatomen oder durch $C_3$-$C_7$-Cycloalkyl und

X für eine Methylengruppe oder eine einfache Bindung steht, wobei $R^2$ ungleich Phenyl oder substituiertes Phenyl ist, wenn X eine einfache Bindung bedeutet,

sowie ihre physiologisch unbedenklichen Säureadditionssalze.

Die neuen Verbindungen haben ein breites und vielseitiges pharmakologisches Wirkungsspektrum und überraschend lange Wirkungsdauer.

Im einzelnen konnten im Tierexperiment folgende Hauptwirkungen nachgewiesen werden:

1. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich nehr oder weniger isoliert in umschriebenen Gefäßge bieten (wie z.B. dem Zentralnervensystem) manifestieren. Die Verbindungen eignen sich daher besonders als Cerebraltherapeutika.

2. Die Verbindungen senken den Blutdruck von normotonen und hypertonen Tieren und können somit als antihypertensiwe Mittel verwendet werden.

Die erfindungsgemäßen Verbindungen eignen sich aufgrund dieser Eigenschaften zur Prophylaxe der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel (z.3. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiums;earat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriuncitrat, Calciumcarbonat und DicaiciumDhosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dorierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und der individuellem Verhalten gegenüber dem Medikament bzw. der Art von dessen Formulierung und dem Zeipunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der

gleiche Dosierungspielraum vorgesehen. Sinngemäß gelten hierbei auch die obigen Ausführungen.

**Beispiel 1**

2-Nitro-1-anilino-1-(1,2 2-trimethylpropyl-amino)-ethen

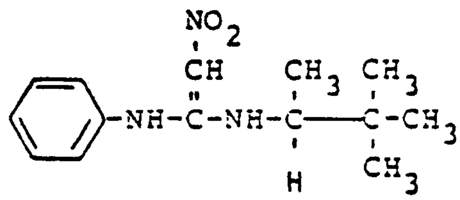

Ein Gemisch aus 10,5 g (0,05 Mol) 2-Nitro-1-anilino-1-methylthioethen und 15,2 g (0,15 Mol) 1,2,2-Trimethylpropylamin wurde 5 Stunden auf 100°C erhitzt. Nach dem Erkalten wurde das Gemisch am Rotationsverdampfer eingeengt und der Rückstand aus Ethanol umkristallisiert.

Fp. 200-202°C

Ausbeute: 9,1 g (69 % d.Th).

Nach dem im Beispiel 1 beschriebenen Verfahren wurden weiterhin hergestellt:

| Beispiel | $R^1$ | X | $R^2$ | Fp. |
|---|---|---|---|---|
| 2 | 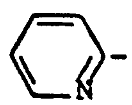 | $CH_2$ | 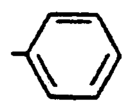 | 176° |
| 3 | 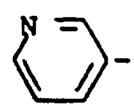 | $CH_2$ | 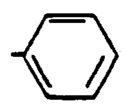 | 213° |
| 4 | 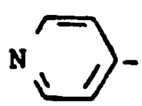 | $CH_2$ | 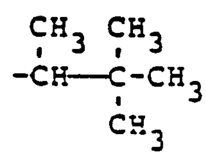 | 185° |

**Fortsetzung der Tabelle**

| Beispiel | $R^1$ | X | $R^2$ | Fp |
|---|---|---|---|---|
| 5 | 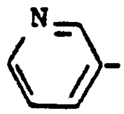 | $CH_2$ | 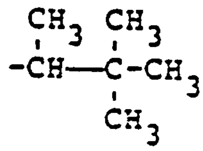 | 189° |
| 6 | 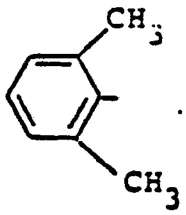 | - | 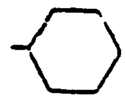 | 241° |
| 7 | 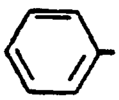 | - | 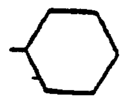 | 191° |
| 8 | 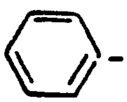 | - | $n-C_6H_{13}$ | 166° |
| 9 | 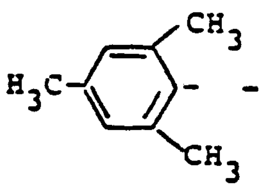 | - | $n-C_6H_{13}$ | 165° |
| 10 | 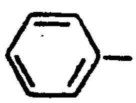 | $CH_2$ | 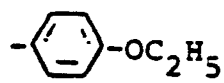 | 222° |
| 11 | 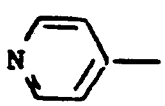 | $CH_2$ | 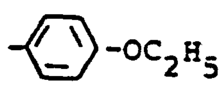 | 201° |
| 12 | 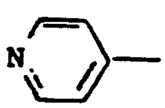 | $CH_2$ | 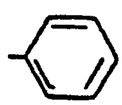 | 207° |
| 13 | 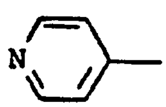 | $CH_2$ | 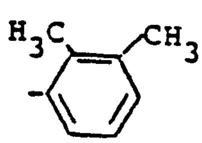 | 194° |

**Fortsetzung der Tabelle**

| Beispiel | $R^1$ | X | $R^2$ | Fp |
|---|---|---|---|---|
| 14 | 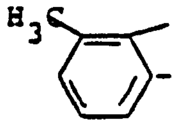 | - | 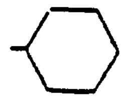 | 162° |
| 15 | 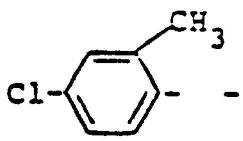 | - | $n-C_6H_{13}$ | 126° |
| 16 | 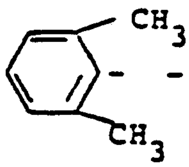 | - | $n-C_6H_{13}$ | 152° |
| 17 | 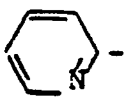 | $CH_2$ | 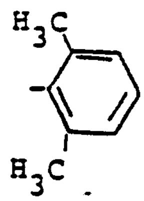 | 189° |
| 18 | 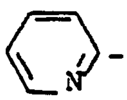 | $CH_2$ | 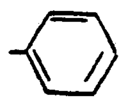 | 176° |
| 19 | 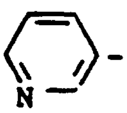 | $CH_2$ | 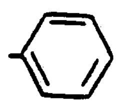 | 213° |
| 20 | 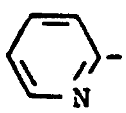 | $CH_2$ | 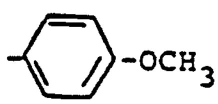 | 172° |
| 21 | 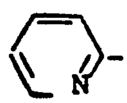 | $CH_2$ | 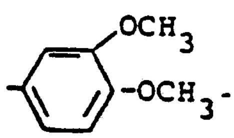 | 166° |

**Fortsetzung der Tabelle**

| Beispiel | $R^1$ | X | $R^2$ | Fp |
|---|---|---|---|---|
| 22 | 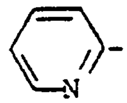 | $CH_2$ | 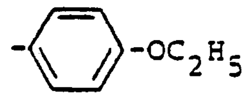 | 183° |
| 23 | 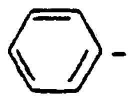 | - | $-CH_2-CH_2-OCH_3$ | 158° |
| 24 | 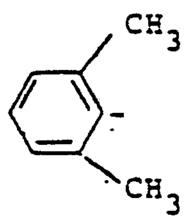 | - | $-CH_2-CH_2-OCH_3$ | 167° |
| 25 | 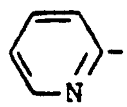 | $CH_2$ | 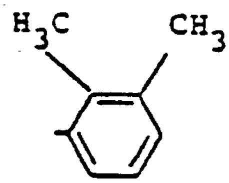 | 174° |
| 26 | 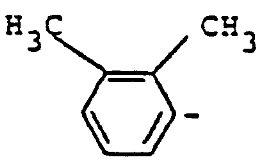 | - | $-CH_2-CH_2-OCH_3$ | 174° |
| 27 | 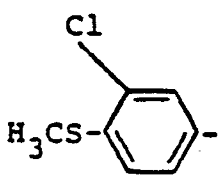 | $CH_2$ | 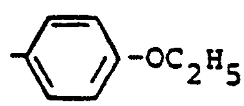 | |
| 28 | 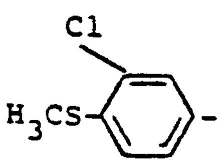 | - | 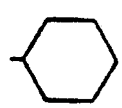 | 176° |

**Fortsetzung der Tabelle**

| Beispiel | $R^1$ | X | $R^2$ | Fp |
|---|---|---|---|---|
| 29 | 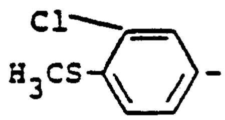 | - | $-CH_2-CH_2-OCH_3$ | 218° |
| 30 | 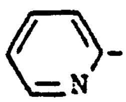 | $CH_2$ | 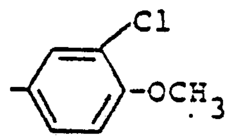 | 197° |
| 31 | 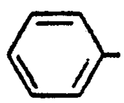 | $CH_2$ | 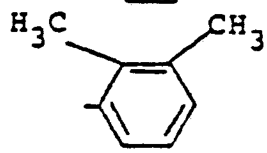 | 186° |
| 32 | 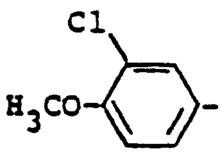 | - | 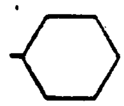 | 189° |
| 33 | 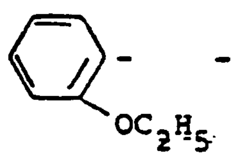 | - | 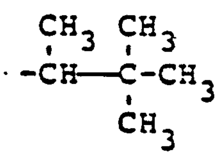 | 197° |
| 34 | 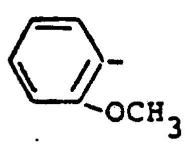 | - | 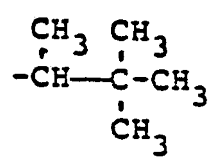 | 205° |
| 35 | 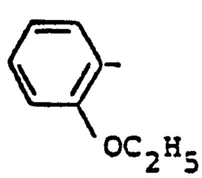 | - | 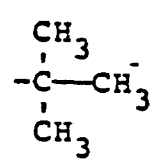 | |

**Fortsetzung der Tabelle**

| Beispiel | $R^1$ | X | $R^2$ | Fp. |
|---|---|---|---|---|
| 36 | 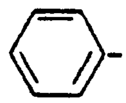 | - | 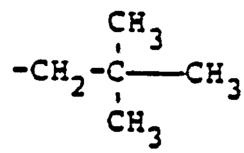 | 197° |
| 37 | 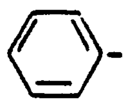 | - | 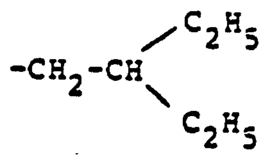 | |
| 38 | 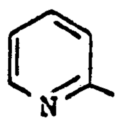 | - | 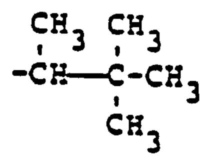 | |
| 39 | 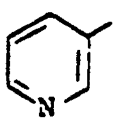 | - | 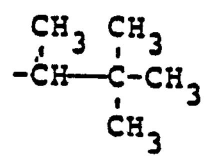 | 182°C |
| 40 | 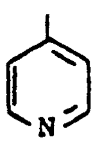 | - | 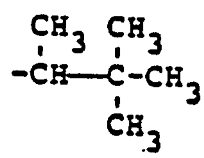 | 110°C |

**Fortsetzung der Tabelle**

| Beispiel | $R^1$ | X | $R^2$ | Fp |
|---|---|---|---|---|
| 41 | 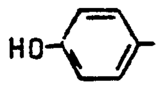 | - | 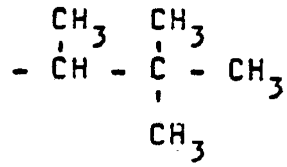 | 243° |
| 42 | 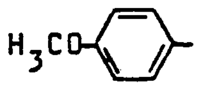 | - | 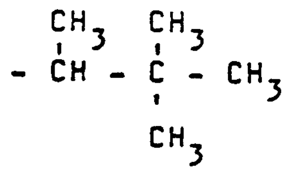 | 229° |
| 43 | 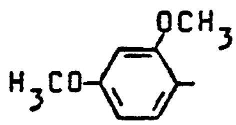 | - | 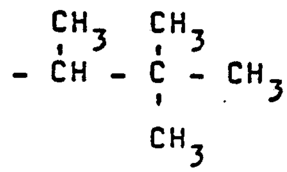 | 233° |
| 44 | 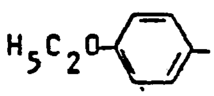 | - | 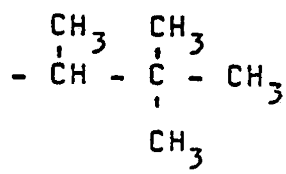 | 207° |
| 45 | 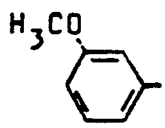 | - | 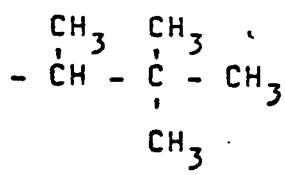 | 168° |
| 46 | 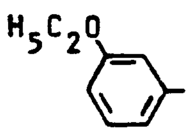 | - | 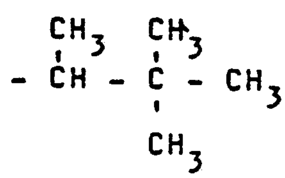 | 164° |
| 47 | 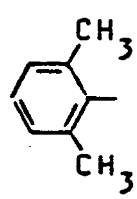 | - | 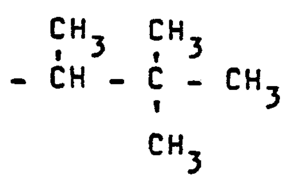 | 236° |

**Patentansprüche**

1. 2-Nitro-1,1-ethendiamine der allgemeinen Formel I

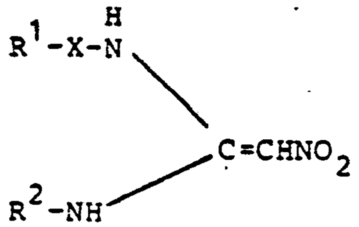

(I)

in welcher

$R^1$ für Phenyl oder Naphthyl steht, welche gegebenenfalls 1- bis 3-mal substituiert sind durch Halogen, Trifluormethyl, Nitro, Cyano, Hydroxy, Amino, Alkylamino, Dialkylamino, Alkyl, Alkoxy oder Alkylmercapto mit jeweils 1 bis 4 C-Atomen in den Alkyl- und Alkoxygruppen oder für Thienyl, Furyl, Pyridyl oder Pyrimidyl steht, welche gegebenenfalls ein- oder zweifach substituiert sind durch Hydroxy, Halogen, Alkyl oder Alkoxy mit jeweils 1 bis 4 C-Atomen,

$R^2$ für geradkettiges oder verzweigtes Alkyl mit bis zu 10 C-Atomen oder für cyclisches Alkyle mit bis zu 8 C-Atomen oder für geradkettiges, verzweigtes oder cyclisches Alkenyl mit 3 bis 8 C-Atomen steht, welche gegebenenfalls substituiert sind durch Alkoxy mit 1 bis 4 C-Atomen oder durch Cycloalkyl mit 3 bis 7 C-Atomen, oder für Phenyl oder Naphthyl steht, welches gegebenenfalls 1- bis 3-fach substituiert ist durch Halogen, Nitro, Cyano, Hydroxy, Trifluormethyl, Amino, Alkylamino, Dialkylamino, Alkyl, Alkoxy oder Alkylmercapto mit jeweils 1 bis 4 C-Atomen in den Alkoxy- und Alkylgruppen, und

X für eine einfache Bindung oder eine Methylengruppe steht, die gegebenenfalls 1- oder 2-fach durch Alkylreste mit 1 bis 4 Kohlenstoffatomen substituiert sein kann, wobei $R^2$ ungleich Phenyl oder substituiertes Phenyl ist, wenn X eine einfache Bindung bedeutet,

sowie ihre physiologisch unbedenklichen Säureadditionssalze.

2. Verbindungen der allgemeinen Formel I gemäß Anspruch 1, in welcher

$R^1$ für Phenyl steht, welches gegebenenfalls 1, 2 oder 3 mal substituiert ist durch gleiche oder verschiedene Substituenten aus der Gruppe Halogen, Trifluormethyl, Alkyl, Alkoxy und Alkylmercapto mit jeweils 1 bis 4 Kohlenstoffatomen, oder für Pyridyl oder Pyrimidyl steht,

$R^2$ für Phenyl steht, welches gegebenenfalls 1- oder 2-fach substituiert ist durch gleiche oder verschiedene Substituenten aus der Gruppe Halogen, Trifluormethyl, Alkyl oder Alkoxy mit jeweils 1 bis 4 Kohlenstoffatomen oder für geradkettiges, verzweigtes oder cyclisches Alkyl mit 1 bis 8 Kohlenstoffatomen oder für geradkettiges, verzweigtes oder cyclisches Alkenyl mit 3 bis 8 Kohlenstoffatomen steht, welches gegebenenfalls substituiert ist durch Alkoxy mit 1 bis 4 Kohlenstoffatomen oder Cycloalkyl mit 3 bis 7 Kohlenstoffatomen, und

X für eine Methylengruppe oder eine einfache Bindung steht, wobei $R^2$ ungleich Phenyl oder substituiertes Phenyl ist wenn X eine einfache Bindung bedeutet.

3. Verfahren zur Herstellung von Verbindungen der allgemeinen Formel I

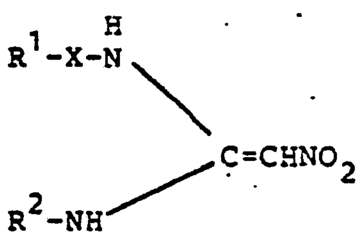

(I)

in welcher

$R^1$ für Phenyl oder Naphthyl steht, welche gegebenenfalls 1- bis 3-mal substituiert sind durch Halogen, Trifluormethyl, Nitro, Cyano, Hydroxy, Amino, Alkylamino, Dialkylamino, Alkyl, Alkoxy oder Alkylmercapto mit jeweils 1 bis 4 C-Atomen in den Alkyl- und Alkoxygruppen oder für Thienyl, Furyl, Pyridyl oder Pyrimidyl steht, welche gegebenenfalls ein- oder zweifach substituiert sind durch Hydroxy, Halogen, Alkyl oder Alkoxy mit

jeweils 1 bis 4 C-Atomen,

$R^2$ für geradkettiges oder verzweigtes Alkyl mit bis zu 10 C-Atomen oder für cyclisches Alkyl mit bis zu 8 C-Atomen oder für geradkettiges, verzweigtes oder cyclisches Alkenyl mit 3 bis 8 C-Atomen steht, welche gegebenenfalls substituiert sind durch Alkoxy mit 1 bis 4 C-Atomen oder durch Cycloalkyl mit 3 bis 7 C-Atomen, oder- für Phenyl oder Naphthyl steht, welches gegebenenfalls 1- bis 3-fach substituiert ist durch Halogen, Nitro, Cyano, Hydroxy, Trifluormethyl, Amino, Alkylamino, Dialkylamino, Alkyl, Alkoxy oder Alkylmercapto mit jeweils 1 bis 4 C-Atomen in den Alkoxy- und Alkylgruppen, und

X für eine einfache Bindung oder eine Methylengruppe steht, die gegebenenfalls 1- oder 2-fach durch Alkylreste mit 1 bis 4 Kohlenstoffatomen substituiert sein kann, wobei $R^2$ ungleich Phenyl oder substituiertes Phenyl ist, wenn X eine einfache Bindung bedeutet,

dadurch gekennzeichnet, daß man Verbindungen der allgemeinen Formel I

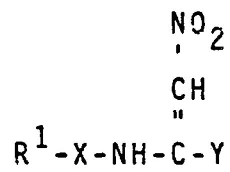

(II)

mit Aminen der allgemeinen Formel III

$R^2$-$NH_2$ (III)

in welcher

$R^1$, $R^2$ und X die oben angegebene Bedeutung haben und

Y für S-Alkyl oder SO-Alkyl mit jeweils 1 bis 8 Kohlenstoffatomen steht,

gegebenenfalls in Gegenwart von inerten organischen Lösungsmitteln bei Temperaturen zwischen 20 und 140°C umsetzt,

oder

Verbindungen der allgemeinen Formel IV

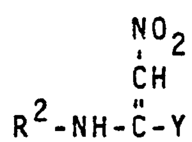

(IV)

mit Aminen der allgemeinen Formel V

$R^1$-X-$NH_2$ (V)

wobei $R^1$, $R^2$ und X die oben angegebene Bedeutung haben und

Y für S-Alkyl oder SO-Alkyl mit 1 bis 10 C-Atomen steht,

gegebenenfalls in Gegenwart von inerten organischen Lösungsmitteln bei Temperaturen zwischen 20 und 140°C umsetzt.

4. Arzneimittel enthaltend mindestens eine Verbindung der allgemeinen Formel I gemäß Anspruch 1.

5. Verfahren zur Herstellung von Arzneimitteln, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.

6. Arzneimittel mit langanhaltender Kreislaufwirkung enthaltend mindestens eine Verbindung der allgemeinen Formel I gemäß Anspruch 1.

7. Verwendung von Verbindungen der allgemeinen Formel I zur Herstellung von Arzneimitteln zur Bekämpfung von Erkrankungen.

**Claims**

1. 2-Nitro-1,1-ethenediamines of the general formula I

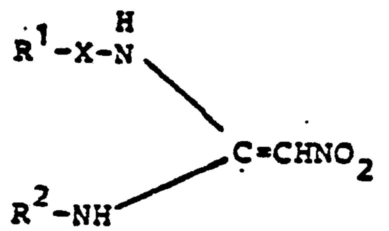

(I)

in which

mono- to trisubstituted by halogen, trifluoromethyl, nitro, cyano, hydroxyl, amino, alkylamino, dialkylamino, alkyl, alkoxy or alkylmercapto with in each case 1 to 4 C atoms in the alkyl and alkoxy groups or represents thienyl, furyl, pyridyl or pyrimidyl, which are optionally mono- or disubstituted by hydroxyl, halogen, alkyl or alkoxy with in each case 1 to 4 C atoms,

$R^2$ represents straight-chain or branched alkyl with up to 10 C atoms or cyclic alkyl with up to 8 C atoms or straightchain, branched or cyclic alkenyl with 3 to 8 C atoms, which are optionally substituted by alkoxy with 1 to 4 C atoms or by cycloalkyl with 3 to 7 C atoms, or represents phenyl or naphthyl which is optionally mono- to trisubstituted by halogen, nitro, cyano, hydroxyl, trifluoromethyl, amino, alkylamino, dialkylamino, alkyl, alkoxy or alkylmercapto with in each case 1 to 4 C atoms in the alkoxy and alkyl groups, and

X represents a single bond or a methylene group which can be optionally mono- or disubstituted by alkyl radicals with 1 to 4 carbon atoms, $R^2$ not being phenyl or substituted phenyl when X denotes a single bond,

and physiologically harmless acid addition salts thereof.

2. Compounds of the general formula I according to Claim 1, in which

$R^1$ represents phenyl which is optionally mono-, dior trisubstituted by identical or different substituents from the group comprising halogen, trifluoromethyl, alkyl, alkoxy and alkylmercapto with in each case 1 to 4 carbon atoms, or represents pyridyl or pyrimidyl,

$R_2$ represents phenyl which is ohtionally mono- or disubstituted by identical or different substituents from the group comprising halogen, trifluoromethyl, alkyl or alkoxy with in each case 1 to 4 carbon atoms, or represents straight-chain, branched or cyclic alkyl with 1 to 8 carbon atoms or straight-chain, branched or cyclic alkenyl with 3 to 8 carbon atoms, which is optionally substituted by alkoxy with 1 to 4 carbon atoms or cycloalkyl with 3 to 7 carbon atoms, and

X represents a methylene group or a single bond, $R^2$ not being phenyl or substituted phenyl when X denotes a single bond.

3. Process for the preparation of compounds of the general formula I

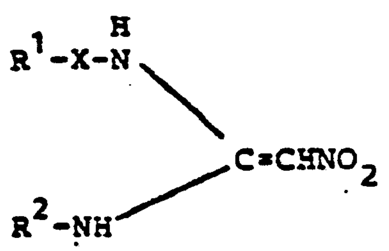

(I)

in which

$R^1$ represents phenyl or naphthyl which are optionally mono- to trisubstituted by halogen, trifluoromethyl, nitro, cyano, hydroxyl, amino, alkylamino, dialkylamino, alkyl, alkoxy or alkylmercapto with in each case 1 to 4 C atoms in the alkyl and alkoxy groups or represents thienyl, furyl, pyridyl or pyrimidyl, which are optionally mono- or disubstituted by hydroxyl, halogen, alkyl or alkoxy with in each case 1 to 4 C atoms,

$R^2$ represnts straight-chain or branched alkul with up to 10 C atoms or cyclic alkyl with up to 6 C atoms or straightchain, branched or cyclic alkenyl with 3 to 8 C atoms, which are optionally substituted by alkoxy with 1 to 4 C atoms or by cycloalkyl with 3 to 7 C atoms, or represents phenyl or naphthyl which is optionally mono- to trisubstituted by halogen nitro cyano hydroxyl, trifluoromethyl, amino, alkylamino, dialkylamino, alkyl, alkoxy or alkylmercapto with in each case 1 to 4 C atoms in the alkoxy and alkyl groups, and

X represents a single bond or a methylene group which can be optionally mono- or disubstituted by alkyl radicals with 1 to 4 carbon atoms, $R^2$ not being phenyl or substituted phenyl when X denotes a single bond, characterised in that compounds of the general formula II

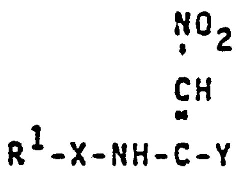

(II)

are reacted with amines of the general formula III

$R^2$-$NH_2$ (III)

in which

$R^1$, $R^2$ and X have the abovementioned meaning and Y represents S-alkyl or SO-alkyl with in each case 1 to 8 carbon atoms,

if appropriate in the presence of inert organic solvents, at temperatures between 20 and 140°C, or compounds of the general formula IV

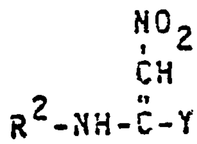

(IV)

are reacted with amines of the general formula V

$R^1$-X-$NH_2$ (V)

wherein

$R^1$, $R^2$ and X have the abovementioned meaning and

Y represents S-alkyl or SO-alkyl with 1 to 10 C atoms, if appropriate in the presence of inert organic solvents, at temperatures between 20 and 140°C.

4. Medicaments containing at least one compound of the general formula I according to Claim 1.

5. Process for the preparation of medicaments, characterised in that compounds of the general formula I according to Claim 1 are converted into a suitable form of administration, if appropriate using customary auxiliaries and excipients.

6. Medicaments which have long-lasting action on the circulation and contain at least one compound of the general formula I according to Claim 1.

7. Use of compounds of the general formula I for the preparation of medicaments for combating diseases.

**Revendications**

1. 2-nitro-1,1-éthènediamines de formule générale I

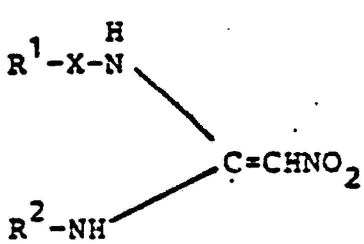

(I)

dans laquelle

$R^1$ représente un groupe phényle ou un groupe naphtyle qui sont éventuellement substitués 1 à 3 fois par des

substituants halogéno, trifluorométhyle, nitro, cyano, hydroxy, amino, alkylamino, dialkylamino, alkyle, alkoxy ou alkylmercapto ayant chacun 1 à 4 atomes de carbone dans les groupes alkyle et alkoxy ou des substituants thiényle, furyle, pyridyle ou pyrimidyle qui sont éventuellement substitués une ou deux fois par des radicaux hydroxy, halogéno, alkyle ou alkoxy ayant chacun 1 à 4 atomes de carbone,

$R^2$ désigne un groupe alkyle à chaîne droite ou ramifié ayant jusqu'à 10 atomes de carbone ou un groupe alkyle cyclique ayant jusqu'à 8 atomes de carbone ou un groupe alcényle chaîne droîte, ramifié ou cyclique ayant 3 à 8 atomes de carbone, qui sont substitués le cas échéant par des substituants alkoxy ayant 1 à 4 atomes de carbone ou cycloalkyle ayant 3 à 7 atomes de carbone, ou bien un groupe phényle ou un groupe naphtyle qui est éventuellement substitué une à trois fois par des substituants halogéno, nitro, cyano, hydroxy, trifluorométhyle, amino, alkylamino, dialkylamino, alkyle, alkoxy ou alkylmercapto ayant dans chaque cas 1 à 4 atomes de carbone dans les groupes alkoxy et alkyle, et

X désigne une liaison simple ou un groupe méthylène qui peut éventuellement être substitué 1 ou 2 fois par des restes alkyle ayant 1 à 4 atomes de carbone, $R^2$ représentant autre chose qu'un groupe phényle ou phényle substitué lorsque X est une liaison simple,

ainsi que leurs sels d'addition d'acidesacceptables du point de vue physiologique.

2. Composés de formule générale I suivant la revendication 1, dans laquelle

$R^1$ représente un groupe phényle qui est éventuellement substitué 1, 2 ou 3 fois par des substituants identiques ou différents choisis dans le groupe des substituants halogéno, trifluorométhyle, alkyle, alkoxy et alkylmercapto ayant chacun 1 à 4 atomes de carbone, ou un groupe pyridyle ou pyrimidyle,

$R^2$ représente un groupe phényle qui est éventuellement substitué 1 ou 2 fois par des substituants identiques ou différents choisis dans le groupe des substituants halogéno, trifluorométhyle, alkyle ou alkoxy ayant chacun 1 à 4 atomes de carbone ou un groupe alkyle à chaîne droite, ramifié ou cyclique ayant 1 à 8 atomes de carbone ou un groupe alcényle à chaîne droite, ramifié ou cyclique ayant 3 à 8 atomes de carbone, qui est éventuellement substitué par un groupe alkoxy ayant 1 à 4 atomes de carbone ou cycloalkyle ayant 3 à 7 atomes de carbone, et

X représente un groupe méthylène ou une liaison simple, $R^2$ étant différent d'un groupe phényle ou phényle substitué lorsque X est une liaison simple.

3. Procédé de production de composés de formule générale I

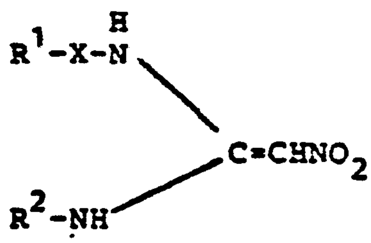

(I)

dans laquelle

$R^1$ représente un groupe phényle ou un groupe naphtyle qui sont éventuellement substitués 1 à 3 fois par des substituants halogéno, trifluorométhyle, nitro, cyano, hydroxy, amino, alkylamino, dialkylamino, alkyle, alkoxy ou alkylmercapto ayant chacun 1 à 4 atomes de carbone dans les groupes alkyle et alkoxy ou des substituants thiényle, furyle, pyridyle ou pyrimidyle qui sont éventuellement substitués une ou deux fois par des radicaux hydroxy, halogéno, alkyle ou alkoxy ayant chacun 1 à 4 atomes de carbone,

$R^2$ désigne un groupe alkyle à chaîne droite ou ramifié ayant jusqu'à 10 atomes de carbone ou un groupe alkyle cyclique ayant jusqu'à 8 atomes de carbone ou un groupe alcényle à chaîne droîte, ramifié ou cyclique ayant 3 à 8 atomes de carbone, qui sont substitués le cas échéant par des substituants alkoxy ayant 1 à 4 atomes de carbone ou cycloalkyle ayant 3 à 7 atomes de carbone, ou bien un groupe phényle ou un groupe naphtyle qui est éventuellement substitué 1 à 3 fois par des substîtuants halogéno, nitro, cyano, hydroxy, trifluorométhyle, amîno, alkylamino, dialkylamino, alkyle, alkoxy ou alkylmercapto ayant dans chaque cas 1 à 4 atomes de carbone dans les groupes alkoxy et alkyle, et

X désigne une liaison simple ou un groupe méthylène qui peut éventuellement être substitué 1 ou 2 fois par des restes alkyle ayant 1 à 4 atomes de carbone, $R^2$ représentant autre chose qu'un groupe phényle ou phényle substitué lorsque X est une liaison simple,

caractérisé en ce qu'on fait réagir des composés de formule générale II

$$\begin{array}{c} NO_2 \\ | \\ CH \\ \| \\ R^1\text{-}X\text{-}NH\text{-}C\text{-}Y \end{array} \qquad (II)$$

avec des amines de formule générale III

$R^2$-$NH_2$ (III)

formules dans lesquelles

$R^1$, $R^2$ et X ont la définition indiquée ci-dessus et Y est un groupe S-alkyle ou SO-alkyle ayant chacun 1 à 8 atomes de carbone,

éventuellement en présence de solvants organiques inertes, à des températures comprises entre 20 et 140°C, ou bien

on fait réagir des composés de formule générale IV

$$\begin{array}{c} NO_2 \\ | \\ CH \\ \| \\ R^2\text{-}NH\text{-}C\text{-}Y \end{array} \qquad (IV)$$

avec des amines de formule générale V

$R^1$-X-$NH_2$ (V)

formules dans lesquelles $R^2$, $R^2$ et X ont les définitions indiquées ci-dessus et

Y est un groupe S-alkyle ou un groupe SO-alkyle ayant 1 à 10 atomes de carbone,

éventuellement en présence de solvants organiques inertes, à des températures comprises entre 20 et 140°C.

4. Médicament contenant au moins un composé de formule générale I suivant la revendication 1.

5. Procédé de préparation de médicaments, caractérisé en ce qu'on transforme des composés de formule générale I suivant la revendication 1, en utilisant le cas échéant des adjuvants et des supports classiques, en une forme d'application appropriée.

6. Médicament à action de longue durée sur la circulation, contenant au moins un composé de formule générale I suivant la revendication 1.

7. Utilisation de composés de formule générale I pour la préparation de médicaments destinés à combattre des maladies.